# EUROPEAN PATENT SPECIFICATION

(11) **EP 4 141 441 B1**
(45) Date of publication and mention of the grant of the patent: **04.03.2026**
(21) Application number: 21792029.7
(22) Date of filing: 12.04.2021
(51) Int. Cl.: G01N 31/22, G01N 33/00, A61J 1/05

(54) **CARBON DIOXIDE DETECTOR**
KOHLENDIOXIDDETEKTOR
DÉTECTEUR DE DIOXYDE DE CARBONE

(30) Priority: 22.04.2020 JP 2020076088
(43) Date of publication of application: 01.03.2023
(73) Proprietor: MITSUBISHI GAS CHEMICAL COMPANY, INC., Chiyoda-ku Tokyo 100-8324 (JP)
(72) Inventor: OKAZAKI, Haruka, Tokyo 125-8601 (JP); ITOU, Yoshiki, Tokyo 125-8601 (JP); OKA, Naoki, Tokyo 125-8601 (JP)
(74) Representative: Hoffmann Eitle
(86) International application number: PCT/JP2021/015150
(87) International publication number: WO 2021/215288

(56) References cited:
- WO-A1-01/44385
- WO-A1-2015/008792
- WO-A1-98/52035
- JP-A- 2003 072 857
- JP-A- 2005 054 048
- JP-A- 2008 224 391
- JP-A- 2008 224 579
- JP-A- 2013 508 676
- JP-A- 2015 219 084
- JP-A- H0 772 138
- JP-A- H08 145 979
- US-A- 5 179 002
- US-A1- 2010 224 508
- US-A1- 2013 236 980
- US-A1- 2016 011 157
- US-A1- 2016 153 946

## Description

### Technical Field

The present invention relates to a carbon dioxide detector.

### Background Art

For preservation of foods, beverages, and chemicals that may deteriorate in contact with oxygen, an inert gas not reacting with the content is enclosed in a packaging container. Carbon dioxide is widely used as a gas used for such applications.

In addition, carbon dioxide is also enclosed for preservation of foods, beverages, and chemicals that may deteriorate in quality or lose medicinal effects due to the release of carbon dioxide.

However, due to damage to the packaging container, the internal carbon dioxide concentration may decrease to result in deterioration of the content.

In particular, a bicarbonate-containing chemical solution is a chemical having properties of releasing carbon dioxide to lose the medicinal effect. Therefore, by packaging a container that accommodates bicarbonate-containing chemical solution together with carbon dioxide in a gas-barrier packaging container, preservation is performed while preventing the release of carbon dioxide. However, in the case of occurrence of a pinhole or a sealing defect resulting from defects in packaging material itself, failure in enclosing of the content, transportation such as distribution, or impacts at home or hospital, the atmosphere in a gas flush package changes, which may cause degeneration of the content, or distribution may be performed without noticing the change in the atmosphere in a gas flush package.

As a simple method to prevent such a situation, a detector that detects carbon dioxide is enclosed together with carbon dioxide in the packaging container, and study has been made to more conveniently and accurately identify the presence of carbon dioxide.

For example, in Patent Literature 1, a carbon dioxide detection agent package having a breathable base material as a package of a detection agent that requires no operation for use, with no risk of scattering the content, is disclosed.

Further, in Patent Literature 2, as carbon dioxide detection agent that causes color transition even in a low-concentration carbon dioxide atmosphere to visually determine the generation of carbon dioxide, a carbon dioxide detection agent including a base material impregnated with an alkaline aqueous solution containing a pH indicator and a water retention agent with adjustment such that the pH indicator exhibits an alkaline color, being enclosed in a pouch having a specific moisture vapor transmission rate, is disclosed.

Further, in Patent Literature 3, as a carbon dioxide detection ink composition capable of easily visually identifying the change in color, a carbon dioxide detection ink containing a pH indicator, a binder and a solvent is disclosed.

Patent Literature 4 relates to an ink composition for sensing carbon dioxide gas containing a pH indicator formed using a combination of two or more types of pH indicator components, binder, and solvent.

Patent Literature 5 relate to a dry reagent detector means for producing a response to the presence of predetermined concentration of carbon dioxide in the sample, said detector means comprising a carrier having an aqueous indicating composition applied thereto, said indicating composition providing an indication of the presence of said predetermined concentration of carbon dioxide in the sample within a diagnostically effective period of time when the carbon dioxide concentration in the sample is at least about 2% and providing said indication after about ten minutes when the carbon dioxide concentration in the sample is about 0.03%.

Patent Literature 6 describes a method comprising associating with a product an indicator composition that changes color after exposure to carbon dioxide, the indicator composition comprising a carbon dioxide attenuator, the indicator composition being sealed from carbon dioxide, and then exposing said indicator composition to air.

Patent Literature 7 relates to a device comprising a visible light emitter circuit configured to provide emitted visible light into a breathing circuit; a first visible light sensor circuit configured to receive a first portion of the emitted visible light; and a processor circuit, coupled to the visible light emitter circuit and to the first visible light sensor circuit, the processor circuit configured to determine a CO₂ level of a respiratory stream in the breathing circuit based on the first portion of the emitted visible light.

Patent Literature 8 is related to an oxygen detecting agent composition comprising polyethylene glycol, a redox dye, a reducing agent, and a basic substance, wherein a content of the polyethylene glycol is 5 to 35% by mass based on a total amount of the oxygen detecting agent composition, the basic substance is a poorly water-soluble basic substance having a solubility in water of less than 1 g/100 g-H₂O at 20° C, and the oxygen detecting agent composition comprising substantially no trisodium phosphate.

### Citation List

### Patent Literature

PTL1: JP 2015-219084 A
PTL2: JP 2008-224579 A
PTL3: International Publication No. WO 2001/044385
PTL4: US 2010/0224508 A1
PTL5: US 5,179,002 A
PTL6: WO 98/52035 A
PTL7: US 2013/0236980 A1
PTL8: US 2016/0153946 A1

### Summary of Invention

### Technical Problem

Along with increasing use and diversification of the shape of packaging containers, various shapes and sizes of carbon dioxide detectors are required. In order to meet the requirement, the detection agent is pulverized and stored in a bag having a size suitable for the packaging container. Although a particulate detection agent is described in Patent Literature 1 and 2, a particulate detection agent having high fluidity is required from the viewpoints of more complicated shapes and productivity. Further, there exists a problem that it takes time to visually identify the change in color of the aqueous solution of the pH indicator soaked in a carrier in a packaging container after the carbon dioxide concentration actually decreases.

Furthermore, in the case where the change in color takes time as described above, time is also required for confirmation of reliable enclosing of carbon dioxide in production of the carbon dioxide detector or in production and packaging of foods or chemicals. Regarding these problems, for example, the carbon dioxide detection agent in Patent Literature 3 is able to determine the leakage of carbon dioxide through color transition when the carbon dioxide concentration decreases, but conversely, unable to identify carbon dioxide generated from the state where there exists no carbon dioxide.

In view of the above, an object of the present invention is to provide a carbon dioxide detector capable of detecting decrease and increase in carbon dioxide concentration in a short time, excellent in fluidity.

### Solution to Problem

As a result of diligent research in view of the above problem, the present inventors have found that a carbon dioxide detector including a carrier impregnated with an ink composition containing a pH indicator, an alkaline agent, a water retention agent and water, containing a specific amount of water, can solve the problem. As a result, the present invention has been completed.

The carbon dioxide detector according to the invention is defined in claim 1.

### Advantageous Effects of Invention

According to the present invention, a carbon dioxide detector capable of detecting decrease and increase in the carbon dioxide concentration in a short time, excellent in fluidity, can be provided.

### Description of Embodiment

### [Carbon dioxide detector]

A carbon dioxide detector of the present invention includes a carrier impregnated with an ink composition containing a pH indicator, an alkaline agent, a water retention agent, and water, and has a water content of 30 to 40 mass%, and wherein the carrier is a porous particle.

### <Ink composition>

The ink composition contained in the carbon dioxide detector contains a pH indicator, an alkaline agent, a water retention agent, and water.

### (pH Indicator)

The pH indicator is used to detect carbon dioxide. Through utilization of the pH change caused by neutralization reaction of alkali with carbon dioxide, carbon dioxide can be clearly detected even in a low concentration region.

The pH indicator used in the carbon dioxide detector of the present invention is preferably an indicator having a transition interval of neutral to alkaline, more preferably an indicator having a transition interval of pH 7.0 to 10.0, and still more preferably an indicator having a transition interval of pH 7.2 to 9.6. Further, an indicator that changes into a clearly different color is preferred to make a clear determination. Further, an indicator having high thermal stability for use under high temperature is preferred.

Examples of the pH indicator having a transition interval from neutral to alkaline include phenol red, cresol red, curcumin, cyanine, α-naphtholphthalein, metacresol purple, thymol blue, o-cresolphthalein, and phenolphthalein. These can be used alone or in combination of two or more. In the case of using in combination, it is preferable to combine those that change into similar color.

Among these, the pH indicator is preferably metacresol purple because the transition interval is alkaline, color transition from purple to yellow is caused at pH 9.0, the change in color is large, and the chemical stability is high.

It is preferable that the amount of the pH indicator be an amount that allows the change in color to be visually identified due to definite coloring, being preferably 0.001 to 0.1 mass%, and more preferably 0.005 to 0.05 mass% in the ink composition. Further, the amount in the carbon dioxide detector is preferably 0.0005 to 0.05 mass%, and more preferably 0.001 to 0.01 mass%.

### (Alkaline agent)

Since carbon dioxide is detected by a pH indicator, the ink composition used in the carbon dioxide detector of the present invention contains an alkaline agent.

It is preferable that the alkaline agent be a compound having high solubility in water.

Examples of the alkaline agent include a metal phosphate, a metal hydroxide, a metal silicate, a metal sulfite, and a metal carbonate. A metal phosphate and a metal hydroxide are preferred, and a metal phosphate is more preferred.

A metal phosphate and a metal hydroxide are preferred due to having particularly high solubility in water.

As the metal phosphate, trisodium phosphate is preferred.

Examples of the metal hydroxide include sodium hydroxide and potassium hydroxide, and sodium hydroxide is preferred.

It is preferable that the amount of the alkaline agent be adjusted according to the pH of the aqueous solution thereof, being preferably 0.02 to 1.0 mass%, more preferably 0.1 to 0.5 mass% in the ink composition. Further, the amount in the carbon dioxide detector is preferably 0.01 to 0.5 mass%, and more preferably 0.05 to 0.2 mass%.

### (Water retention agent)

In order to retain water, the ink composition used for the carbon dioxide detector of the present invention contains a water retention agent. The type of the water retention agent is not particularly limited as long as it has an effect of lowering the water activity when added to the carbon dioxide detector, and the following may be suitably used.

The water retention agent is preferably at least one selected from the group consisting of a polyhydric alcohol, a polyalkylene glycol, an acrylic polymer and cellulose, and more preferably a polyhydric alcohol.

Examples of the polyhydric alcohol include glycerin, ethylene glycol, and propylene glycol, and in particular, glycerin is preferred.

Examples of the polyalkylene glycol include polyethylene glycol.

Examples of the acrylic polymer include a polyacrylic acid salt and a polyacrylic acid ester.

The amount of the water retention agent is preferably 10 to 70 mass%, more preferably 20 to 60 mass%, in the ink composition from the viewpoints of rapidly detecting the carbon dioxide concentration and effectively reducing the water activity. Further, the amount in the carbon dioxide detector is preferably 5 to 50 mass%, and more preferably 10 to 40 mass%.

### (Water)

The water content is preferably 40 to 75 mass%, and more preferably 50 to 65 mass% in the ink composition from the viewpoint of rapidly detecting the carbon dioxide concentration. Further, the water content in the carbon dioxide detector is 30 to 40 mass%, preferably 32 to 40 mass%, more preferably 34 to 40 mass%, still more preferably 36 to 40 mass%, and furthermore preferably 36 to 39.5 mass%.

The mass ratio of water to the water retention agent (water/ water retention agent) is preferably 1.0 to 2.0, more preferably 1.2 to 1.9, from the viewpoint of rapidly detecting the carbon dioxide concentration. With a mass ratio of water to the water retention agent controlled to the range, a uniform carbon dioxide detector can be obtained in production of the carbon dioxide detector. In particular, the sufficiently mixing can be achieved to make a uniform particulate carbon dioxide detector.

The mass ratio of water to the carrier described below (water/carrier) is preferably 0.5 to 2.0, more preferably 0.7 to 1.0, from the viewpoint of rapidly detecting the carbon dioxide concentration and improving the fluidity. With a mass ratio of water to the carrier controlled to the range, color development is improved and filling into a packaging material can be easily performed.

### <Carrier>

The carbon dioxide detector of the present invention includes a carrier impregnated with the ink composition. By impregnating a carrier and forming into a particle shape, excellent fluidity can be obtained.

As the carrier, porous particles are used, and in particular, porous inorganic particles are preferred.

Examples of the porous inorganic particles include silica gel, diatomaceous earth, zeolite, and porous silicates, and porous silicates are preferred.

Among the porous silicates, magnesium aluminometasilicate is preferred due to suitability for use in the fields of foods and pharmaceuticals.

The carrier is preferably colorless or white so that the change in color is clearly identified. The pH of the carrier is preferably neutral to alkaline.

The average particle size of the carrier is preferably 1 to 500 µm, more preferably 100 to 300 µm, from the viewpoints of fluidity and compounding properties.

From the viewpoint of fluidity, the shape of the carrier is preferably spherical or approximately spherical, and more preferably spherical.

The amount of the carrier is preferably 20 to 70 mass%, more preferably 30 to 60 mass%, in the carbon dioxide detector, from the viewpoints of rapidly detecting the carbon dioxide concentration and improving the fluidity.

### <Spreading agent>

It is preferable that the carbon dioxide detector of the present invention contain a spreading agent from the viewpoint of improving fluidity while maintaining the definiteness of the change in color.

It is preferable that the spreading agent adheres to the outer surface of the carrier impregnated with the ink composition.

The spreading agent is preferably a spherical fine particle, more preferably silica, and still more preferably hydrophobic silica.

The amount of the spreading agent is preferably 0.1 to 5 mass%, more preferably 0.2 to 2 mass% in the carbon dioxide detector from the viewpoint of improving the fluidity.

### <Packaging>

The carbon dioxide detector of the present invention is in a particle form with high fluidity, and preferably packed for preservation and use, though being usable as it is depending on the intended use.

Examples of the packaging form include a bag shape and a box shape, and a bag shape is preferred.

As the material used for packaging, it is preferable to use a transparent resin film in order to visually identify the change in color from the outside for determination of the presence or absence of carbon dioxide.

Preferred examples of the packaging material include biaxially stretched polypropylene (OPP), unstretched polypropylene (CPP), low density polyethylene (LDPE), linear low density polyethylene (LLDPE), high density polyethylene (HDPE), polyethylene terephthalate (PET), polyvinyl alcohol (PVA), polyvinyl chloride (PVC), polyvinylidene chloride (PVDC), and stretched polyamide (ONY).

Further, since it is necessary to open a part of the package for contact with an external atmosphere for detection of the carbon dioxide concentration, it is preferable to provide a ventilation hole in a part of the package. It is preferable that the vent allow carbon dioxide to pass through while not allowing the carbon dioxide detector to pass through, being preferably a vent formed of filamentous material or fine porous film, more preferably a vent formed of filamentous material. Examples of the porous film include nonwoven fabric.

A part of the filamentous material exists inside the package (a portion for accommodating the carbon dioxide detector), and another part thereof reaches outside the package. In the case where the filamentous material forms a vent, breathability is achieved through a space between fibers that constitute the filamentous material, or a space between the fibers and a material used for packaging.

The filamentous material may be an aggregate of fibrous material in a filamentous shape, preferably having a melting point of 80°C or more so as to withstand molding of a package. As an example of the filamentous material, a sewing thread is preferably used. Examples of the substance of filamentous material include polyethylene terephthalate, cotton, polyester, vinylon, silk and nylon, and polyethylene terephthalate, and polyethylene terephthalate, nylon and vinylon are preferred. As the thickness of the thread, a yarn count of 1 to 100 is preferred, a yarn count of 10 to 80 is more preferred, and a yarn count of 15 to 60 is still more preferred.

### [Method for producing carbon dioxide detector]

The method for producing the carbon dioxide detector of the present invention is not limited. It is preferable that the carbon dioxide detector is produced by impregnating a carrier with an ink composition containing a pH indicator, an alkaline agent, a water retention agent and water, and it is more preferable that the carbon dioxide detector is produced by impregnating a carrier with an ink composition containing a pH indicator, an alkaline agent, a water retention agent and water, followed by mixing with a spreading agent. The method according to the invention for producing a carbon dioxide detector is defined in claim 8.

In order to uniformly impregnate the carrier with the ink composition, it is preferable that stirring be performed using a mixer.

Further, in order to allow the spreading agent to uniformly adhere around the carrier, it is preferable that stirring be performed using a mixer.

As the conditions for stirring, it is preferable that stirring be performed at a speed of 10 to 40 rpm.

### [Package]

The package of the present invention includes the carbon dioxide detector disposed inside an external package having a carbon dioxide-containing gas enclosed therein. In other words, the package of the present invention includes a carbon dioxide detector including a carrier impregnated with an ink composition containing a pH indicator, an alkaline agent, a water retention agent, and water, having a water content of 30 to 40 mass% and wherein the carrier is a porous particle, which is disposed in an external package having a carbon dioxide-containing gas enclosed therein.

The package of the present invention includes a carbon dioxide-containing gas and the carbon dioxide detector inside the package, that is, inside an external package, and also includes a content such as chemicals and foods in the external package.

It is preferable that the content such as chemicals and foods be further accommodated in a container disposed inside the external package.

It is preferable that the external package used in the package of the present invention be made of a gas barrier material through which a carbon dioxide-containing gas hardly passes. Specific examples of the material include a resin film of ethylene-vinyl alcohol copolymer, polyethylene terephthalate or nylon, and a composite film composed of the resin film on which silica, alumina, or the like is vapor-deposited. However, a part of the external package is transparent in order to visually identify the change in color of the carbon dioxide detector from outside the external package.

The carbon dioxide-containing gas may be composed of carbon dioxide alone, or may be composed of carbon dioxide and an inert gas. The concentration of carbon dioxide in the carbon dioxide-containing gas is preferably 1 to 50%.

Examples of the content disposed in the package of the present invention include chemicals and foods, and specific examples thereof include chemicals containing a hydrogen carbonate such as sodium hydrogen carbonate, fruits and vegetables, raw meat, and confectionery. In particular, in the case where a bicarbonate-containing infusion fluid is used as the chemicals, due to detection of the carbon dioxide concentration in a short time by the carbon dioxide detector, product deterioration can be preferably prevented by early use or by moving into a carbon dioxide atmosphere for preservation.

### [Method for preserving bicarbonate-containing infusion fluid]

The method for preserving a bicarbonate-containing infusion fluid of the present invention includes disposing the carbon dioxide detector and a bicarbonate-containing infusion fluid inside an external package having a carbon dioxide-containing gas enclosed therein. In other words, the method for preserving a bicarbonate-containing infusion fluid of the present invention includes disposing a carrier, the carrier being a porous particle, impregnated with an ink composition containing a pH indicator, an alkaline agent, a water retention agent and water, having a water content of 30 to 40 mass%, and a bicarbonate-containing infusion fluid, in an external package having a carbon dioxide-containing gas enclosed therein.

The bicarbonate-containing infusion fluid is used as an extracellular fluid replenisher for compensating an extracellular fluid using bicarbonate ions as alkalizing agent. The bicarbonate-containing infusion fluid has properties of releasing carbon dioxide to lose the medicinal effect. Therefore, by packaging the container containing the bicarbonate-containing infusion fluid together with carbon dioxide in a gas barrier packaging container, the release of carbon dioxide is prevented to achieve preservation.

In the case where the bicarbonate-containing infusion fluid is preserved in a carbon dioxide-containing gas, use of the carbon dioxide detector allows decrease in the concentration of carbon dioxide in the gas to be detected in a short time, so that the bicarbonate-containing infusion fluid, which tends to be easily deteriorated, can be used up in an early stage, or can be preserved again in a carbon dioxide atmosphere. Thus, the deterioration of the bicarbonate-containing infusion fluid can be efficiently prevented.

As the external package used in the preservation method of the present invention, it is preferable to use an external package that can be suitably used for the package described above. Specific examples thereof include a resin film of ethylene-vinyl alcohol copolymer, polyethylene terephthalate and nylon, and a composite film composed of the resin film on which silica, alumina, or the like is vapor-deposited.

The carbon dioxide-containing gas is preferably composed of carbon dioxide and nitrogen gas. The concentration of carbon dioxide in the carbon dioxide-containing gas is preferably 1 to 50%, more preferably 1 to 15%, still more preferably 3 to 10%.

The preservation temperature in the preservation method of the present invention is preferably 5 to 40°C.

The preservation method of the present invention exhibits a higher effect when moving or transporting a bicarbonate-containing infusion fluid. In other words, a method for moving or transporting a bicarbonate-containing infusion fluid including disposing the carbon dioxide detector and a bicarbonate-containing infusion fluid in an external package having a carbon dioxide-containing gas enclosed therein is also preferred.

In the case where the bicarbonate-containing infusion fluid is moved or transported, the external package is easily subject to damage that cannot be visually identified due to external stimulus or impact from the outside. Use of the carbon dioxide detector allows decrease in the concentration of carbon dioxide to be detected in a short time, so that the bicarbonate-containing infusion fluid in a damaged external package can be used up in an early stage, or can be preserved again in a carbon dioxide atmosphere. Thus, the deterioration of the bicarbonate-containing infusion fluid can be efficiently prevented.

### Examples

Hereinafter, the present invention will be described in more detail with reference to Examples and Comparative Examples, though the present invention is not limited to these Examples.

### [Evaluation]

The carbon dioxide detectors obtained in Examples and Comparative Examples were evaluated as follows.

### <Evaluation of carbon dioxide concentration detection (detection time/change in color)>

The detection of carbon dioxide concentration was evaluated as follows.

### (Preparation of carbon dioxide detector package)

In a bag (with ventilation threads) made of transparent laminated film of OPP/LLDPE with a size of 2.5 cm by 3.0 cm, 0.2 g of the carbon dioxide detector obtained in Examples and Comparative Examples was placed in, and the opening was hermetically heat-sealed to obtain a package of the carbon dioxide detector.

### (Test method)

### [1] Increase in concentration of carbon dioxide

The package of the carbon dioxide detector was placed in a bag made of gas barrier film having a capacity of 250 mL, and a mixed gas of 5% carbon dioxide and 95% nitrogen was enclosed therein. Subsequently, the opening of the gas barrier film bag was hermetically heat-sealed, and the bag was preserved in an environment at 25°C. The colors of the detector immediately after preservation (after 0 hour), after 1.5 hours, and after 3 hours were evaluated by the following method.

### [2] Decrease in concentration of carbon dioxide

The package of the carbon dioxide detector was placed in a bag made of gas barrier film having a capacity of 250 mL, and a mixed gas of 5% carbon dioxide and 95% nitrogen was enclosed therein. Subsequently, the opening of the gas barrier film bag was hermetically heat-sealed, and the bag was preserved in an environment at 25°C for 3 hours. The package of the carbon dioxide detector was then taken out, preserved under atmosphere at 25°C (carbon dioxide concentration: 0.04%), and the colors of the detector immediately after preservation (after 0 hour), after 15 hours, and after 32 hours were evaluated by the following method.

### (Evaluation method)

### (1) Δb* value

The b* value of the carbon dioxide detector was measured from outside of the package of the carbon dioxide detector using a color difference meter, and the difference from the b* value immediately after preservation (after 0 hour) was determined as Δb* value. The larger the Δb* value, the larger the change in color, so that the detectability of the carbon dioxide concentration is more excellent.

### (2) Color by color chart

The color of the carbon dioxide detector was visually evaluated from the outside of the package of the carbon dioxide detector by a color chart (manufactured by G&E, New Color Databook). The greater the change in color from the color immediately after preservation (after 0 hour), the better the detectability of carbon dioxide concentration.

In the evaluations (1) and (2), the shorter the time until the change in color occurs, the shorter the time required for detection of the change in carbon dioxide concentration.

### <Fluidity>

The fluidity was evaluated as follows.

The angle of repose of the carbon dioxide detector was measured with a powder measuring instrument (PT-X manufactured by Hosokawa Micron Co., Ltd.). The smaller the angle of repose, the better the fluidity.

### [Carbon dioxide detector]

### Example 1

Into 83 g of distilled water, 0.016 g of metacresol purple as pH indicator, 0.25 g of trisodium phosphate dodecahydrate as alkaline agent, and 59.5 g of glycerin as water retention agent were mixed to obtain a purple ink composition. Magnesium aluminometasilicate (trade name: Neucilin SG2, average particle size: about 200 µm) in amount of 100 g as carrier was impregnated with 142.7 g of the ink composition, and 0.75 g of hydrophobic silica was added thereto. The mixture was stirred to obtain a purple carbon dioxide detector. The evaluation results are shown in Table 1.

### Examples 2 to 3 and Comparative Examples 1 to 2

A purple carbon dioxide detector was obtained in the same manner as in Example 1, except that the amount of distilled water was changed as shown in Table 1. The evaluation results are shown in Table 1.

### Example 4

A purple carbon dioxide detector was obtained in the same manner as in Example 1, except that no hydrophobic silica was used. The evaluation results are shown in Table 1.

**Table 1**

| | | | Example 1 | | Example 2 | | Example 3 | | Example 4 | | Comparative Example 1 | | Comparative Example 2 | |
|---|---|---|---|---|---|---|---|---|---|---|---|---|---|---|
| Carbon oxide detector | | | (g) | (mass%) | (g) | (mass%) | (g) | (mass%) | (g) | (mass%) | (g) | (mass%) | (g) | (mass%) |
| | Mg aluminometasilicate | | 100 | 41.065 | 100 | 38.533 | 100 | 43.008 | 100 | 41.192 | 100 | 45.763 | 100 | 35.649 |
| | Metacresol purple | | 0.016 | 0.007 | 0.016 | 0.006 | 0.016 | 0.007 | 0.016 | 0.007 | 0.016 | 0.007 | 0.016 | 0.006 |
| | Trisodium phosphate dodecahydrate | | 0.25 | 0.103 | 0.25 | 0.096 | 0.25 | 0.108 | 0.25 | 0.103 | 0.25 | 0.114 | 0.25 | 0.089 |
| | Glycerin | | 59.5 | 24.434 | 59.5 | 22.927 | 59.5 | 25.590 | 59.5 | 24.509 | 59.5 | 27.229 | 59.5 | 21.211 |
| | Hydrophobic silica | | 0.75 | 0.308 | 0.75 | 0.289 | 0.75 | 0.323 | 0.00 | 0.000 | 0.75 | 0.343 | 0.75 | 0.267 |
| | Water | | 83 | 34.084 | 99.0 | 38.148 | 72 | 30.966 | 83 | 34.189 | 58 | 26.543 | 120 | 42.778 |
| | Water/carrier | | 0.83 | | 0.99 | | 0.72 | | 0.83 | | 0.58 | | 1.2 | |
| | Water/water retention agent | | 1.4 | | 1.7 | | 1.2 | | 1.4 | | 1.0 | | 2.0 | |
| Evaluation of carbon dioxide concentration detection (1) Δb* value | | | b* value | Δb* value') | b* value | Δb* value') | b* value | Δb* value') | b* value | Δb* value²) | b* value | Δb* value¹⁾ | b* value | Δb* value¹⁾ |
| | Increase in carbon dioxide concentration: Violet (0.04%) to Yellow (5%) | 0h | -7 | 0 | -12 | 0 | -6 | 0 | -6 | 0 | -4 | 0 | -13 | 0 |
| | | 1.5h | 2 | 9 | 4 | 16 | 4 | 10 | 2 | 8 | 4 | 8 | 1 | 14 |
| | | 3h | 9 | 16 | 7 | 19 | 8 | 14 | 9 | 15 | 6 | 10 | 6 | 19 |
| | Decrease in carbon dioxide concentration: Yellow (5%) to Violet (0.04%) | 0h | 8 | 0 | 11 | 0 | 7 | 0 | 8 | 0 | 6 | 0 | 10 | 0 |
| | | 15h | -3 | 11 | -5 | 16 | -3 | 10 | -3 | 11 | -2 | 8 | -8 | 18 |
| | | 32h | -6 | 14 | -8 | 19 | -5 | 12 | -5 | 13 | -3 | 9 | -12 | 22 |
| Evaluation of carbon dioxide concentration detection (2) Color chart | Increase in carbon dioxide concentration: Violet (0.04%) to Yellow (5%) | 0h | 0962 | | 0964 | | 0961 | | 0962 | | 0961 | | 0964 | |
| | | 3h | 0097 | | 0130 | | 0097 | | 0097 | | 0097 | | 0130 | |
| | Decrease in carbon dioxide concentration: Yellow (5%) to Violet (0.04%) | 0h | 0145 | | 0130 | | 0145 | | 0145 | | 0145 | | 0130 | |
| | | 15h | 1009 | | 1058 | | 1009 | | 1009 | | 0145 | | 1010 | |
| Evaluation of fluidity | Angle of response | ° | 23 | | 22 | | 21 | | 29 | | 20 | | 33 | |

| | | | | | | | | | | | | | | |
|---|---|---|---|---|---|---|---|---|---|---|---|---|---|---|
| 1) Δb* value=b* value (after X hours)-b* value (after 0 hour) | | | | | | | | | | | | | | |

As shown in Table 1, the carbon dioxide detector in Comparative Example 1 had a low sensitivity to the decrease in carbon dioxide concentration, and in particular, no change was observed even after 15 hours in the evaluation by the color chart. Further, the carbon dioxide detector in Comparative Example 2 had a large angle of repose and was inferior in fluidity. On the other hand, it can be seen that the carbon dioxide detector in Examples can detect the decrease and increase in the carbon dioxide concentration in a short time and is excellent in fluidity.

## Claims

1. A carbon dioxide detector comprising a carrier impregnated with an ink composition comprising a pH indicator, an alkaline agent, a water retention agent, and water, wherein the carrier is a porous particle, **characterized in that** the carbon dioxide detector has a water content of 30 to 40 mass%.

2. The carbon dioxide detector according to claim 1, wherein the pH indicator is metacresol purple.

3. The carbon dioxide detector according to claim 1 or 2, wherein the water retention agent is at least one selected from the group consisting of a polyhydric alcohol, a polyalkylene glycol, an acrylic polymer and cellulose.

4. The carbon dioxide detector according to any one of the claims 1 to 3, wherein the mass ratio of water to the carrier (water/carrier) is 0.7 to 1.0.

5. The carbon dioxide detector according to any one of claims 1 to 4, wherein the mass ratio of water to the water retention agent (water/water retention agent) is 1.2 to 1.9.

6. The carbon dioxide detector according to any one of claims 1 to 5, further comprising a spreading agent.

7. The carbon dioxide detector according to claim 6, wherein the spreading agent is hydrophobic silica.

8. A method for producing a carbon dioxide detector according to claim 6 or 7, comprising impregnating a carrier with an ink composition comprising a pH indicator, an alkaline agent, a water retention agent and water, followed by mixing with a spreading agent.

9. A package comprising the carbon dioxide detector according to any one of claims 1 to 7 disposed inside an external package having a carbon dioxide-containing gas enclosed therein.

10. A method for preserving a bicarbonate-containing infusion fluid, comprising disposing the carbon dioxide detector according to any one of claims 1 to 7 and the bicarbonate-containing infusion fluid in an external package having a carbon dioxide-containing gas enclosed therein.

## Patentansprüche

1. Kohlendioxiddetektor, umfassend einen Träger, der mit einer Tintenzusammensetzung imprägniert ist, die einen pH-Indikator, ein alkalisches Mittel, ein Wasserrückhaltemittel und Wasser umfasst,
wobei der Träger ein poröses Partikel ist,
**dadurch gekennzeichnet, dass** der Kohlendioxiddetektor einen Wassergehalt von 30 bis 40 Masse-% aufweist.

2. Kohlendioxiddetektor gemäß Anspruch 1, wobei der pH-Indikator Metacresolpurpur ist.

3. Kohlendioxiddetektor gemäß Anspruch 1 oder 2, wobei das Wasserrückhaltemittel mindestens eines, ausgewählt aus der Gruppe, bestehend aus einem mehrwertigen Alkohol, einem Polyalkylenglykol, einem Acrylpolymer und Cellulose, ist.

4. Kohlendioxiddetektor gemäß einem der Ansprüche 1 bis 3, wobei das Massenverhältnis von Wasser zu dem Träger (Wasser/Träger) 0,7 bis 1,0 beträgt.

5. Kohlendioxiddetektor gemäß einem der Ansprüche 1 bis 4, wobei das Massenverhältnis von Wasser zu dem Wasserrückhaltemittel (Wasser/Wasserrückhaltemittel) 1,2 bis 1,9 beträgt.

6. Kohlendioxiddetektor gemäß einem der Ansprüche 1 bis 5, ferner umfassend ein Spreitmittel.

7. Kohlendioxiddetektor gemäß Anspruch 6, wobei das Spreitmittel hydrophobes Silika ist.

8. Verfahren zur Herstellung eines Kohlendioxiddetektors gemäß Anspruch 6 oder 7, umfassend das Imprägnieren eines Trägers mit einer Tintenzusammensetzung, die einen pH-Indikator, ein alkalisches Mittel, ein Wasserrückhaltemittel und Wasser umfasst, gefolgt von Mischen mit einem Spreitmittel.

9. Verpackung, umfassend den Kohlendioxiddetektor gemäß einem der Ansprüche 1 bis 7, der in einer externen Verpackung mit einem darin eingeschlossenen kohlendioxidhaltigen Gas angeordnet ist.

10. Verfahren zur Konservierung einer bicarbonathaltigen Infusionsflüssigkeit, umfassend das Anordnen des Kohlendioxiddetektors gemäß einem der Ansprüche 1 bis 7 und der bicarbonathaltigen Infusionsflüssigkeit in einer externen Verpackung mit einem darin eingeschlossenen kohlendioxidhaltigen Gas.

## Revendications

1. Détecteur de dioxyde de carbone comprenant un support imprégné d'une composition d'encre comprenant un indicateur de pH, un agent alcalin, un agent de rétention d'eau et de l'eau,
dans lequel le support est une particule poreuse,
**caractérisé en ce que** le détecteur de dioxyde de carbone présente une teneur en eau allant de 30 à 40 % en masse.

2. Détecteur de dioxyde de carbone selon la revendication 1, dans lequel l'indicateur de pH est le pourpre de métacrésol.

3. Détecteur de dioxyde de carbone selon la revendication 1 ou la revendication 2, dans lequel l'agent de rétention d'eau est au moins un sélectionné dans le groupe consistant en un polyalcool, un polyalkylène glycol, un polymère acrylique et de la cellulose.

4. Détecteur de dioxyde de carbone selon l'une quelconque des revendications 1 à 3, dans lequel le rapport massique de l'eau au support (eau/support) va de 0,7 à 1,0.

5. Détecteur de dioxyde de carbone selon l'une quelconque des revendications 1 à 4, dans lequel le rapport massique de l'eau à l'agent de rétention d'eau (eau/agent de rétention d'eau) va de 1,2 à 1,9.

6. Détecteur de dioxyde de carbone selon l'une quelconque des revendications 1 à 5, comprenant en outre un agent de diffusion.

7. Détecteur de dioxyde de carbone selon la revendication 6, dans lequel l'agent de diffusion est de la silice hydrophobe.

8. Procédé de production d'un détecteur de dioxyde de carbone selon la revendication 6 ou la revendication 7, comprenant le fait d'imprégner un support par une composition d'encre comprenant un indicateur de pH, un agent alcalin, un agent de rétention d'eau et de l'eau, suivi du fait de mélanger avec un agent de diffusion.

9. Emballage comprenant le détecteur de dioxyde de carbone selon l'une quelconque des revendications 1 à 7 disposé à l'intérieur d'un emballage externe dans lequel est enfermé un gaz contenant du dioxyde de carbone.

10. Procédé de conservation d'une solution de perfusion contenant du bicarbonate, comprenant le fait de disposer le détecteur de dioxyde de carbone selon l'une quelconque des revendications 1 à 7 et de la solution de perfusion contenant du bicarbonate dans un emballage externe dans lequel est enfermé un gaz contenant du dioxyde de carbone.
